# EUROPÄISCHE PATENTANMELDUNG

(11) **EP 4 506 028 A1**
(43) Veröffentlichungstag der Anmeldung: **12.02.2025**
(21) Anmeldenummer: 24187315.7
(22) Anmeldetag: 09.07.2024
(51) Int. Cl.: A61M 25/00

(54) **INFUSIONSSYSTEM**

(30) Priorität: 03.08.2023 DE 102023120613
(71) Anmelder: B. Braun Melsungen AG, 34212 Melsungen (DE)
(72) Erfinder: Sauer, Holger, 45731 Waltrop (DE); Schröder, Tobias, 34212 Melsungen (DE); Wildhagen, Jens, 30659 Hannover (DE)
(74) Vertreter: Patentanwälte Ruff, Wilhelm, Beier, Dauster & Partner mbB

(57) **Zusammenfassung**

Infusionssystem (1) zur Verwendung bei einer Schmerztherapie, aufweisend einen Katheter (100), wenigstens einem Lumen (103), mit mehreren Auslässen (A), wobei die mehreren Auslässe (A) jeweils radial durch eine Katheterwandung (106) des Katheters (100) erstreckt und proximodistal voneinander beabstandet sind, wobei die mehreren Auslässe (A) wenigstens einen ersten Auslass (104) und wenigstens einen zweiten Auslass (105) aufweisen, wobei der erste Auslass (104) unabhängig von einem in dem Lumen (103) vorherrschenden Fluiddruck (P) zur Abgabe eines Schmerzmittels (S) geöffnet ist, und wobei der zweite Auslass (105) zur fluiddruckabhängigen Abgabe des Schmerzmittels (S) eingerichtet und in Abhängigkeit des innerhalb des Lumens (103) vorherrschenden Fluiddrucks (P) überführbar ist zwischen einem Öffnungszustand, in welchem der zweite Auslass (105) zur Abgabe des Schmerzmittels (S) geöffnet ist, und einem Schließzustand, in welchem der zweite Auslass (105) geschlossen ist.

## Beschreibung

Die Erfindung betrifft ein Infusionssystem zur Verwendung bei einer Schmerztherapie, aufweisend einen Katheter mit einem proximalen Ende, einem distalen Ende, wenigstens einem Lumen, das zwischen dem proximalen Ende und dem distalen Ende längserstreckt ist, und mit mehreren Auslässen, wobei die mehreren Auslässe jeweils mit dem wenigstens einen Lumen fluidleitend verbunden sind, und wobei die mehreren Auslässe jeweils radial durch eine Katheterwandung des Katheters erstreckt und proximodistal voneinander beabstandet sind.

Ein derartiges Infusionssystem ist im Bereich der Medizintechnik allgemein bekannt und wird im Rahmen einer Schmerztherapie zur Abgabe eines Schmerzmittels verwendet. Um das Schmerzmittel an mehreren Stellen verteilt abgeben zu können, verfügt der Katheter über mehrere proximodistal voneinander beabstandet angeordnete Auslässe. Dabei gibt die Positionierung der Auslässe die örtliche Verteilung des Schmerzmittels vor.

Aufgabe der Erfindung ist es, ein Infusionssystem der eingangs genannten Art bereitzustellen, das eine verbesserte Schmerztherapie ermöglicht.

Diese Aufgabe wird dadurch gelöst, dass die mehreren Auslässe wenigstens einen ersten Auslass und wenigstens einen zweiten Auslass aufweisen, wobei der erste Auslass unabhängig von einem in dem Lumen vorherrschenden Fluiddruck zur Abgabe eines Schmerzmittels geöffnet ist, und wobei der zweite Auslass zur fluiddruckabhängigen Abgabe des Schmerzmittels eingerichtet und in Abhängigkeit des innerhalb des Lumens vorherrschenden Fluiddrucks überführbar ist zwischen einem Öffnungszustand, in welchem der zweite Auslass zur Abgabe des Schmerzmittels geöffnet ist, und einem Schließzustand, in welchem der zweite Auslass geschlossen ist. Die erfindungsgemäße Lösung erlaubt eine druckabhängige Steuerung der Abgabe des Schmerzmittels durch den wenigstens zweiten Auslass. Da die mehreren Auslässe und damit der wenigstens eine erste Auslass und der wenigstens eine zweite Auslass proximodistal, d.h. entlang der Längsachse des Katheters, voneinander beabstandet sind, kann hierdurch die Position der Schmerzmittelabgabe beeinflusst werden. Dabei geht die Erfindung von der Überlegung aus, dass eine über die Länge des Katheters steuerbare Schmerzmittelabgabe aus medizinischer Sicht vorteilhaft sein kann. Die erfindungsgemäße Lösung erlaubt eine solche Steuerung, indem die Schmerzmittelabgabe durch den wenigstens einen zweiten Auslass in Abhängigkeit des in dem wenigstens einen Lumen vorherrschenden Fluiddrucks gesteuert werden kann. Zu diesem Zweck ist der wenigstens eine zweite Auslass zur fluiddruckabhängigen Abgabe des Schmerzmittels eingerichtet. Bei einer Ausgestaltung ist der wenigstens eine zweite Auslass zu diesem Zweck speziell gestaltet, beispielsweise in Form eines Schlitzes oder dergleichen. Bei einer weiteren Ausgestaltung ist dem wenigstens einen zweiten Auslass ein Ventil zur druckabhängigen Steuerung zugeordnet. Im Unterschied zu dem wenigstens einen zweiten Auslass ist der wenigstens eine erste Auslass nicht steuerbar, insbesondere stets geöffnet. Der wenigstens eine zweite Auslass ist in dem Schließzustand, insbesondere vollständig, geschlossen. Bei einer Ausgestaltung ist der wenigstens eine zweite Auslass in dem Schließzustand nicht vollständig geschlossen, so dass gegenüber dem Öffnungszustand eine Schmerzmittelabgabe mit verringerter Flussrate erfolgt. Vorzugsweise ist der wenigstens eine zweite Auslass derart gestaltet, dass eine in dem Öffnungszustand durch den wenigstens einen zweiten Auslass abgegebene Flussrate einer Flussrate durch den wenigstens einen ersten Auslass entspricht.

In Ausgestaltung der Erfindung ist der zweite Auslass durch einen Schlitz in der Katheterwandung gebildet, wobei die Katheterwandung wenigstens im Bereich des Schlitzes elastisch nachgiebig ist, wobei der Schlitz bei einem ersten Fluiddruck geschlossen ist, und wobei der Schlitz bei einem höheren zweiten Fluiddruck infolge einer durch den zweiten Fluiddruck verursachten elastischen Deformation der Katheterwandung geöffnet ist. Durch die Ausbildung des zweiten Auslasses als Schlitz wird die fluiddruckbedingte Steuerbarkeit mit besonders einfachen Mitteln erreicht. Der Schlitz reicht radial von einer dem Lumen zugewandten Innenseite der Katheterwandung bis auf eine der Umgebung zugewandte Außenseite der Katheterwandung. Durch die wenigstens im Bereich des Schlitzes elastisch nachgiebige Gestaltung der Katheterwandung kann sich der Schlitz infolge einer fluiddruckbedingten elastischen Deformation der Katheterwandung öffnen und schließen. Liegt der erste Fluiddruck an, ist der Schlitz geschlossen und die Katheterwandung ist im Bereich des Schlitzes im Wesentlichen nicht deformiert. Liegt der zweite Fluiddruck an, der im Vergleich zu dem ersten Fluiddruck höher ist, erfolgt eine elastische Deformation der Katheterwandung im Bereich des Schlitzes, wodurch sich dieser öffnet und eine Abgabe des Schmerzmittels durch den Schlitz ermöglicht. Bei einer Ausgestaltung ist der Schlitz durchgängig gerade längserstreckt, beispielsweise in proximodistaler Längsrichtung des Katheters oder quer zu der Längsrichtung, insbesondere in Umfangsrichtung. Bei einer weiteren Ausgestaltung ist der Schlitz gekrümmt längserstreckt. Bei einer weiteren Ausgestaltung weist der Schlitz mehrere unterschiedlich orientierte und miteinander verbundene Schlitzabschnitte auf. Durch eine Wahl der Länge und/oder Formgebung des Schlitzes sowie die elastischen Eigenschaften der Katheterwandung im Bereich des Schlitzes kann das Öffnungs- und Schließverhalten mit einfachen Mitteln an unterschiedliche Erfordernisse angepasst werden. Durch die elastische Gestaltung der Katheterwandung wenigstens im Bereich des Schlitzes kehrt dieser bei einer Verringerung des Fluiddrucks selbsttätig von dem Öffnungs- in den Schließzustand zurück.

In weiterer Ausgestaltung der Erfindung ist der Schlitz proximodistal längserstreckt und in dem Öffnungszustand in Umfangsrichtung der Katheterwandung geweitet. Vorzugsweise ist der Schlitz durchgängig gerade längserstreckt. Durch die proximodistal längserstreckte Ausrichtung des Schlitzes kann ein verbessertes Öffnungs- und Schließverhalten in Abhängigkeit des vorherrschenden Fluiddrucks erreicht werden. Dies insbesondere im Vergleich zu einer grundsätzlich denkbaren Ausrichtung des Schlitzes in Umfangsrichtung der Katheterwandung.

In weiterer Ausgestaltung der Erfindung ist der Schlitz in radialer Blickrichtung U-förmig in die Katheterwandung eingebracht und berandet einen Wandabschnitt, der in dem Öffnungszustand relativ zu der übrigen Katheterwandung radial nach außen ausgestellt ist. Der Wandabschnitt funktioniert nach Art einer elastischen Lasche oder auch Klappe. Liegt der erste Fluiddruck an, ist der Wandabschnitt bündig zu der übrigen Katheterwandung, wodurch der Schlitz geschlossen ist. Liegt der höhere zweite Fluiddruck an, ist der Wandabschnitt infolge der Druckeinwirkung radial nach außen ausgestellt, wodurch der Schlitz geöffnet ist. Durch die wenigstens im Bereich des Schlitzes elastisch nachgiebige Gestaltung der Katheterwandung kehrt der Wandabschnitt bei einer Verminderung des Drucks ausgehend von dem Öffnungsselbsttätig in den Schließzustand zurück.

In weiterer Ausgestaltung der Erfindung ist dem zweiten Auslass ein Ventil zugeordnet, wobei das Ventil zum fluiddruckabhängigen Öffnen und Schließen eingerichtet ist, wobei das Ventil bei einem ersten Fluiddruck geschlossen und bei einem höheren zweiten Fluiddruck geöffnet ist. Das Ventil kann jede für den vorliegenden Zweck geeignete Bauform aufweisen. Das Ventil öffnet und schließt in Abhängigkeit des Fluiddruckes selbststätig, beispielsweise nach Art eines Rückschlagventils.

In weiterer Ausgestaltung der Erfindung ist das Ventil dem zweiten Auslass stromaufwärts vorgeschaltet. Beispielsweise kann das Ventil in dem Lumen oder einem dem zweiten Auslass zugordneten Abzweig des Lumens angeordnet sein. Bei einer weiteren Ausgestaltung ist das Ventil dem zweiten Auslass stromabwärts nachgeschaltet.

In weiterer Ausgestaltung der Erfindung ist der wenigstens eine erste Auslass proximodistal, insbesondere zentral, zwischen wenigstens zwei zweiten Auslässen angeordnet. Die beiden zweiten Auslässe sind somit in Bezug auf die Längsachse des Katheters beidseits des wenigstens einen ersten Auslasses angeordnet. In diesem Zusammenhang kann auch von einer peripheren Anordnung der wenigstens zwei zweiten Auslässe gesprochen werden. Diese Ausgestaltung erlaubt eine Steuerung der Schmerzmittelabgabe in einem peripheren Bereich. Demgegenüber erfolgt die Abgabe im Bereich des ersten Auslasses zwischen den wenigstens zwei zweiten Auslässen unabhängig von dem vorherrschenden Fluiddruck, insbesondere stetig. Bei einer alternativen Ausgestaltung ist der wenigstens eine zweite Auslass proximodistal, insbesondere zentral, zwischen den wenigstens zwei ersten Auslässen angeordnet. Bei dieser Ausgestaltung erfolgt eine peripher in Abhängigkeit des Fluiddruckes unveränderliche Schmerzmittelabgabe, wohingegen in einem zentralen Bereich zwischen den zwei ersten Auslässen eine fluiddruckgesteuerte Abgabe durch den wenigstens einen zweiten Auslass erfolgen kann.

In weiterer Ausgestaltung der Erfindung ist eine Gruppe mit mehreren, insbesondere exakt drei, ersten Auslässen proximodistal, insbesondere zentral, zwischen zwei Gruppen mit jeweils mehreren, insbesondere exakt drei, zweiten Auslässen angeordnet. Durch eine gruppenweise Anordnung mit jeweils mehreren ersten oder zweiten Auslässen kann die örtliche Verteilung der Schmerzmittelabgabe in verbesserter Weise gesteuert werden. Bei einer alternativen Ausgestaltung ist eine Gruppe mit mehreren, insbesondere exakt drei, zweiten Auslässen proximodistal, insbesondere zentral, zwischen zwei Gruppen mit jeweils mehreren, insbesondere exakt drei, zweiten Auslässen angeordnet.

In weiterer Ausgestaltung der Erfindung sind eine Fördereinrichtung und eine Steuereinrichtung vorhanden, wobei die Fördereinrichtung fluidleitend mit dem wenigstens einen Lumen des Katheters verbunden und zum Fördern des Schmerzmittels durch das wenigstens eine Lumen mit unterschiedlichen Fluiddrücken eingerichtet ist, und wobei die Steuereinrichtung mit der Fördereinrichtung verbunden und zum Steuern des Fluiddrucks und damit zum Öffnen und/oder Schließen des wenigstens einen zweiten Auslasses eingerichtet ist. Die Fördereinrichtung kann jede für den vorliegenden Zweck geeignete Bauform aufweisen. Beispielsweise kann die Fördereinrichtung eine Kolben-, Peristaltik- oder Elastomerpumpe sein, wobei selbstverständlich auch andere Bauformen denkbar und möglich sind. Die Steuereinrichtung ist zum Steuern der Fördereinrichtung eingerichtet, wobei im Speziellen eine Steuerung des Fluiddrucks vorgesehen ist. Durch die Steuerung des Fluiddrucks kann der wenigstens eine zweite Auslass des Katheters mittels der Steuereinrichtung geöffnet und/oder geschlossen werden. Das auf diese Weise gesteuerte Öffnen und/oder Schließen des wenigstens einen zweiten Auslasses erlaubt eine besonders einfach örtliche Anpassung der Schmerzmittelabgabe.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Ansicht eine Ausführungsform eines erfindungsgemäßen Infusionssystems mit einem Katheter, einer Fördereinrichtung und einer Steuereinrichtung,
- Fig. 2, 3: einen Abschnitt des Katheters in radialer Blickrichtung im Bereich eines zweiten Auslasses, wobei der zweite Auslass in Abhängigkeit eines Fluiddrucks einen geschlossenen Zustand (Fig. 2) und einen geöffneten Zustand einnimmt (Fig. 3),
- Fig. 4, 5: den in den Fig. 2 und 3 gezeigten Bereich des Katheters mit einer alternativen Gestaltung des zweiten Auslasses in geschlossenem Zustand (Fig. 4) und in geöffnetem Zustand (Fig. 5), wobei Fig. 5 einen Längsschnitt mit einer um 90° gedrehten radialen Blickrichtung zeigt, und
- Fig. 6: ein Funktionsdiagramm zur Verdeutlichung einer weiteren Ausgestaltung.

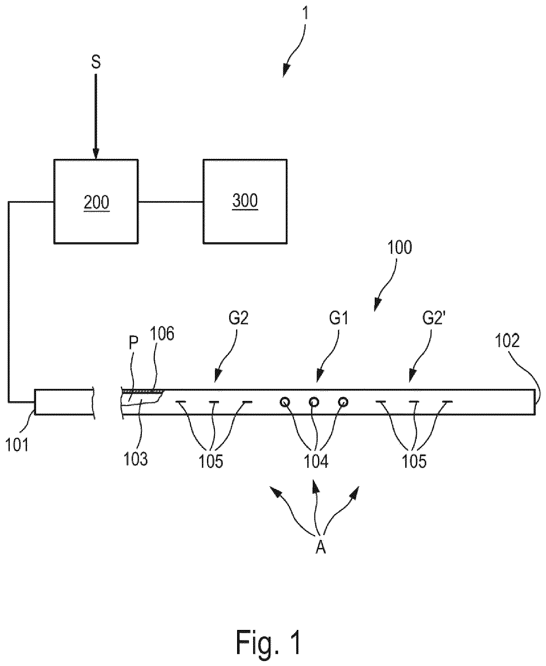

Gemäß Fig. 1 ist ein Infusionssystem 1 zur Verwendung bei einer Schmerztherapie vorgesehen und weist einen Katheter 100, eine Fördereinrichtung 200 und eine Steuereinrichtung 300 auf. Die Fördereinrichtung 200 und/oder die Steuereinrichtung 300 sind optional und nicht bei sämtlichen Ausführungsformen vorhanden.

Der Katheter 100, der auch als Schmerzkatheter bezeichnet werden kann, weist ein proximales Ende 101, ein distales Ende 102, wenigstens ein Lumen 103 sowie mehrere Auslässe A auf.

Das proximale Ende 101 und das distale Ende 102 sind proximodistal voneinander beabstandet. Der Katheter 100 ist zwischen dem proximalen Ende 101 und dem distalen Ende 102 axial längserstreckt. Die in Fig. 1 gezeigte durchgängig gerade Längserstreckung und die gezeigte Formgebung sind als schematisch vereinfacht und rein exemplarisch zu verstehen.

Das Lumen 103 ist zwischen dem proximalen Ende 101 und dem distalen Ende 102 längserstreckt. Bei in den Figuren nicht gezeigten Ausführungsformen weist der Katheter mehrere Lumen auf.

Die mehreren Auslässe A dienen der Abgabe eines Schmerzmittels S. Das Schmerzmittel S ist mittels der Fördereinrichtung 200, die auf nicht näher gezeigte Weise fluidleitend an das proximale Ende 101 angeschlossen ist, in das Lumen 103 und von diesem durch die Auslässe A förderbar. Die Auslässe A sind jeweils mit dem Lumen 103 fluidleitend verbunden. Bei einer in den Figuren nicht gezeigten Ausführungsform sind unterschiedliche Auslässe mit unterschiedlichen Lumen des Katheters fluidleitend verbunden. Weiter sind die Auslässe A vorliegend jeweils radial durch eine Katheterwandung 106 des Katheters 100 erstreckt und proximodistal voneinander beabstandet. Mit anderen Worten sind die Auslässe A in Längsrichtung des Katheters 100 verteilt angeordnet. Diese verteilte Anordnung der Auslässe A ermöglicht eine örtlich verteilte Abgabe des Schmerzmittels S.

Die in Fig. 1 gezeigte über die Länge des Katheters 100 verteilte Anordnung der mehreren Auslässe A sowie deren Anzahl sind als rein exemplarisch zu verstehen. Bei in den Figuren nicht gezeigten Ausführungsformen ist eine unterschiedliche Anzahl und/oder Anordnung der mehreren Auslässe vorgesehen. Im einfachsten Fall weist der Katheter 100 exakt zwei Auslässe auf.

Die mehreren Auslässe A umfassen wenigstens einen ersten Auslass 104 und wenigstens einen zweiten Auslass 105.

Bei der gezeigten Ausführungsform sind mehrere erste Auslässe 104 und mehrere zweite Auslässe 105 vorhanden. Zur vereinfachten Beschreibung wird nachfolgend lediglich auf einen/den ersten Auslass 104 und einen/den zweiten Auslass 105 Bezug genommen. Das zu dem (einen) ersten Auslass 104 Gesagte gilt sinngemäß auch für die weiteren ersten Auslässe. Das zu dem (einen) zweiten Auslass 105 Gesagte gilt sinngemäß auch für die weiteren zweiten Auslässe.

Der erste Auslass 104 ist bei der gezeigten Ausführungsform stets geöffnet, wodurch eine permanente Abgabe des Schmerzmittels S durch den ersten Auslass 104 erfolgen kann.

Der zweite Auslass 105 ist zur gesteuerten Abgabe des Schmerzmittels S eingerichtet. Zu diesem Zweck ist der zweite Auslass 105 in Abhängigkeit eines innerhalb des Lumens 103 vorherrschenden Fluiddrucks P überführbar zwischen einem Öffnungszustand und einem Schließzustand. In dem Öffnungszustand ist der zweite Auslass 105 zur Abgabe des Schmerzmittels S geöffnet. In dem Schließzustand ist der zweite Auslass 105 geschlossen. Bei der gezeigten Ausführungsform ist der zweite Auslass in dem Schließzustand vollständig geschlossen, so dass keine oder jedenfalls keine praktisch relevante Abgabe des Schmerzmittels S erfolgen kann. Bei einer in den Figuren nicht gezeigten Ausführungsform ist der zweite Auslass in dem Schließzustand teilweise und/oder unvollständig geschlossen, so dass eine Abgabe des Schmerzmittels S mit einer gegenüber dem Öffnungszustand verringerter Flussrate erfolgen kann.

Durch das von dem Fluiddruck P abhängige Öffnen und Schließen des zweiten Auslasses 105 kann die Abgabe des Schmerzmittels S örtlich gesteuert werden. In geschlossenem Zustand des zweiten Auslasses 105 erfolgt die Abgabe lediglich durch den ersten Auslass 104. In geöffnetem Zustand des zweiten Auslasses 105 erfolgt die Abgabe des Schmerzmittels durch den ersten Auslass 104 und durch den zweiten Auslass 105.

Der Fluiddruck P wird mittels der Fördereinrichtung 200 generiert und mittels der Steuereinrichtung 300 gesteuert. Durch die Steuerung des Fluiddrucks P erfolgt demnach mittelbar eine Steuerung der örtlichen Schmerzmittelabgabe.

Die Fördereinrichtung 200 und die Steuereinrichtung 300 sind in Fig. 1 schematisch dargestellt. Die Fördereinrichtung 200 ist an eine nicht näher gezeigte Quelle des Schmerzmittels S angeschlossen. Die Fördereinrichtung 200 kann jede für den vorliegenden Zweck geeignete Bauweise haben. Beispielsweise kann die Fördereinrichtung 200 als Kolbenpumpe, Peristaltikpumpe oder Elastomerpumpe ausgeführt sein.

Bei der gezeigten Ausführungsform ist eine Gruppe G1 mit mehreren ersten Auslässen 104 vorhanden. Zudem sind zwei Gruppen G2, G2' mit jeweils mehreren zweiten Auslässen 105 vorhanden. Bei der gezeigten Ausführungsform weisen die Gruppen G1, G2, G2` jeweils exakt drei erste Auslässe 104 bzw. exakt drei zweite Auslässe 105 auf. Bei einer in den Figuren nicht gezeigten Ausführungsform sind hiervon unterschiedliche Anzahlen vorgesehen. Die Gruppe G1 mit den ersten Auslässen 104 ist proximodistal zwischen den zwei Gruppen G2, G2' mit den zweiten Auslässen 105 angeordnet. Diese Anordnung ist vorliegend zentral, so dass auch von einer zentralen Gruppe G1 und zwei peripheren Gruppen G2, G2` gesprochen werden kann. Die in Fig. 1 gezeigte Ausführungsform erlaubt eine Steuerung der peripheren Schmerzmittelabgabe, wohingegen die Schmerzmittelabgabe in dem zentralen Bereich der Gruppe G1 nicht in Abhängigkeit des Fluiddrucks steuerbar ist.

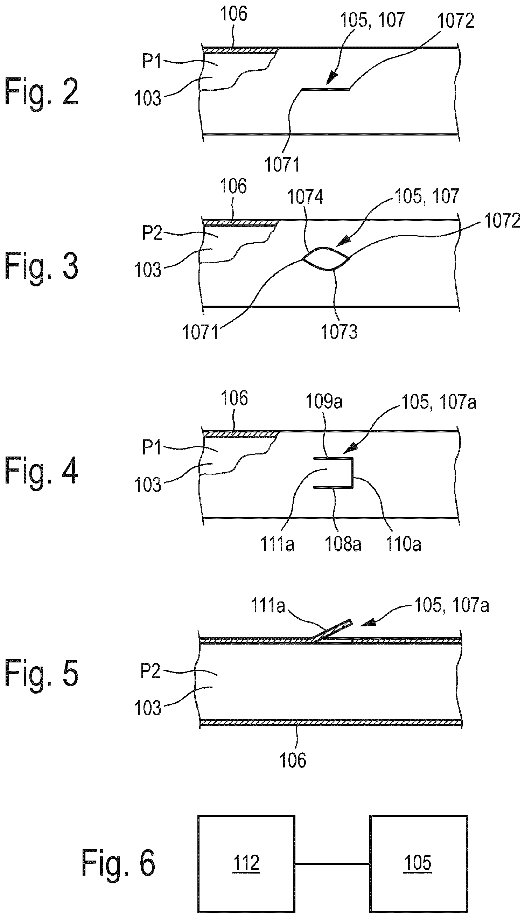

Die Fig. 2 bis 6 zeigen Varianten unterschiedlicher Gestaltungen des zweiten Auslasses 105. Die gezeigten Gestaltungen lassen jeweils eine fluiddruckabhängige Steuerung des zweiten Auslasses 105 zwischen dem Öffnungs- und dem Schließzustand zu. Die in den Fig. 2 bis 6 gezeigten Gestaltungen können alternativ oder in Kombination vorhanden sein. Beispielsweise können die zweiten Auslässe 105 der Gruppe G2 eine unterschiedliche Gestaltung aufweisen als die mehreren zweiten Auslässe 105 der Gruppe G2`.

Bei der in den Fig. 2 und 3 gezeigten Variante ist der zweite Auslass 105 durch einen Schlitz 107 gebildet. Die Katheterwandung 105 ist wenigstens im Bereich des Schlitzes 107 elastisch nachgiebig. Liegt ein erster Fluiddruck P1 innerhalb des Lumens 103 an ist der Schlitz 107 geschlossen (Fig. 2), d.h. der zweite Auslass 105 nimmt den Schließzustand ein. Liegt innerhalb des Lumens 103 ein im Vergleich zu dem ersten Fluiddruck P1 höherer zweiter Fluiddruck P2 an, ist der Schlitz 107 infolge einer durch den zweiten Fluiddruck P2 verursachten elastischen Deformation der Katheterwandung 106 geöffnet (siehe Fig. 3). Sinkt der Fluiddruck ausgehend von dem zweiten Fluiddruck P2 ab, schließt der Schlitz 107 sich infolge der elastischen Eigenschaften der Katheterwandung 106 selbsttätig.

Vorliegend ist die Katheterwandung 106 aus einem elastischen Kunststoff gefertigt. Infrage kommt jeder für den vorliegenden Zweck geeignete Kunststoff mit elastischen Eigenschaften und Eignung für medizinische Anwendungen. Solche Kunststoffe sind dem Fachmann bekannt.

Bei der in den Fig. 2 und 3 gezeigten Variante ist der Schlitz 107 proximodistal längserstreckt. Die Längserstreckung ist gerade und parallel zur Längsachse des Katheters 100. Der Schlitz 107 ist zwischen einem proximalen Schlitzende 1071 und einem distalen Schlitzende 1072 durchgängig gerade längserstreckt. In radialer Richtung erstreckt sich der Schlitz ausgehend von einer dem Lumen 103 zugewandten Innenseite (ohne Bezugszeichen) der Katheterwandung 106 bis auf eine der Umgebung zugewandte Außenseite (ohne Bezugszeichen) der Katheterwandung 106. In dem Öffnungszustand (Fig. 3) ist der Schlitz 107 in Umfangsrichtung der Katheterwandung 106 geweitet. In diesem geweiteten Zustand sind in Umfangsrichtung einander gegenüberliegende Innenflächen 1073, 1074 des Schlitzes 107 voneinander abgehoben, wodurch der Auslass 105 freigegeben wird. In dem Schließzustand (Fig. 2) liegen die Innenflächen 1073, 1074 fluiddicht aufeinander, d.h. der zweite Auslass 105 ist geschlossen.

Bei der in den Fig. 4 und 5 gezeigten Variante ist der Schlitz 107a in der radialen Blickrichtung der Fig. 4 U-förmig in die Katheterwandung 106 eingebracht. Der U-förmige Schlitz 107a berandet einen Wandabschnitt 111a der Katheterwandung 106. Der Wandabschnitt 111a ist in dem Öffnungszustand (Fig. 5) relativ zu der übrigen Katheterwandung 106 radial nach außen ausgestellt oder aufgeklappt. Hierdurch wird der zweite Auslass 105 freigegeben. In dem Schließzustand (Fig. 4) liegt der Wandabschnitt 111a im Wesentlichen flächenbündig mit der übrigen Katheterwandung 106, so dass der zweite Auslass 105 geschlossen ist. Der Wandabschnitt 111a bildet eine Art Klappe oder Lasche, die in Abhängigkeit der vorherrschenden Fluiddrucks relativ zu der umgebenden Katheterwandung 106 elastisch beweglich ist. Liegt der zweite Fluiddruck P2 an, federt der Wandabschnitt 111 a zum Freigeben des zweiten Auslasses 105 elastisch nach außen. Wird der Fluiddruck ausgehend von dem zweiten Fluiddruck P2 abgesenkt, federt der Wandabschnitt 111a aufgrund der elastischen Eigenschaften der Katheterwandung 106 selbsttätig in den geschlossenen Zustand zurück.

Bei der in den Fig. 4 und 5 gezeigten Variante weist der Schlitz 107a einen ersten Schlitzabschnitt 108a, einen zweiten Schlitzabschnitt 109a und einen dritten Schlitzabschnitt 110a auf. Der erste Schlitzabschnitt 108a und der zweite Schlitzabschnitt 109a sind jeweils proximodistal längserstreckt und in Umfangsrichtung voneinander beabstandet. Vorliegend sind der erste Schlitzabschnitt 108a und der zweite Schlitzabschnitt 109a parallel. Zudem sind der erste Schlitzabschnitt 108a und der zweite Schlitzabschnitt 109a gleich lang. Der dritte Schlitzabschnitt 110a ist in Umfangsrichtung längserstreckt und jeweils an einem distalen Ende der beiden Schlitzabschnitte 108a, 109a angeordnet. Der dritte Schlitzabschnitt 110a verbindet ein distales Ende (ohne Bezugszeichen) des ersten Schlitzabschnitts 108a mit einem distalen Ende (ohne Bezugszeichen) des zweiten Schlitzabschnitts 109a. An einem dem dritten Schlitzabschnitt 110a gegenüberliegenden Ende des ersten Schlitzabschnitts 108a und des zweiten Schlitzabschnitts 109a ist der von dem U-förmigen Schlitz 107a berandete Wandabschnitt 111a mit der übrigen Katheterwandung 106a verbunden. Diese Verbindung bildet eine elastisches Festkörpergelenk oder elastisches Scharnier, wobei die gedachte Gelenkachse vorliegend parallel zur Umfangsrichtung der Katheterwandung 106 orientiert ist.

Bei der in Fig. 6 gezeigten Variante ist dem zweiten Auslass 105 ein Ventil 112 zugeordnet. In Fig. 6 sind der zweite Auslass 105 und das Ventil 112 schematisch vereinfacht dargestellt. Das Ventil 112 ist zum fluiddruckabhängigen Öffnen und Schließen eingerichtet und geschlossen, sofern der ersten Fluiddruck P1 anliegt, und geöffnet, sofern der zweite Fluiddruck P2 anliegt. Das Ventil 112 kann auch als Zweiwegeventil bezeichnet werden. Das Ventil 112 kann jede für den vorliegenden Zweck geeignete Bauform aufweisen und dem zweiten Auslass 105 entweder stromaufwärts vorgeschaltet oder stromabwärts nachgeschaltet sein. Im Übrigen ist es denkbar, dass das Ventil 112 selbst den zweiten Auslass 105 bildet oder umfasst.

## Patentansprüche

1. Infusionssystem (1) zur Verwendung bei einer Schmerztherapie, aufweisend einen Katheter (100) mit einem proximalen Ende (101), einem distalen Ende (102), wenigstens einem Lumen (103), das zwischen dem proximalen Ende (101) und dem distalen Ende (102) längserstreckt ist, und mit mehreren Auslässen (A), wobei die mehreren Auslässe (A) jeweils mit dem wenigstens einen Lumen (103) fluidleitend verbunden sind, und wobei die mehreren Auslässe (A) jeweils radial durch eine Katheterwandung (106) des Katheters (100) erstreckt und proximodistal voneinander beabstandet sind,
**dadurch gekennzeichnet, dass** die mehreren Auslässe (A) wenigstens einen ersten Auslass (104) und wenigstens einen zweiten Auslass (105) aufweisen,
wobei der erste Auslass (104) unabhängig von einem in dem Lumen (103) vorherrschenden Fluiddruck (P) zur Abgabe eines Schmerzmittels (S) geöffnet ist, und
wobei der zweite Auslass (105) zur fluiddruckabhängigen Abgabe des Schmerzmittels (S) eingerichtet und in Abhängigkeit des innerhalb des Lumens (103) vorherrschenden Fluiddrucks (P) überführbar ist zwischen einem Öffnungszustand, in welchem der zweite Auslass (105) zur Abgabe des Schmerzmittels (S) geöffnet ist, und einem Schließzustand, in welchem der zweite Auslass (105) geschlossen ist.

2. Infusionssystem (1) nach Anspruch 1, **dadurch gekennzeichnet, dass** der zweite Auslass (105) durch einen Schlitz (107, 107a) in der Katheterwandung (106) gebildet ist, wobei die Katheterwandung (106) wenigstens im Bereich des Schlitzes (107, 107a) elastisch nachgiebig ist, wobei der Schlitz (107, 107a) bei einem ersten Fluiddruck (P1) geschlossen ist, und wobei der Schlitz (107, 107a) bei einem höheren zweiten Fluiddruck (P2) infolge einer durch den zweiten Fluiddruck (P2) verursachten elastischen Deformation der Katheterwandung (106) geöffnet ist.

3. Infusionssystem (1) nach Anspruch 2, **dadurch gekennzeichnet, dass** der Schlitz (107) proximodistal längserstreckt und in dem Öffnungszustand in Umfangsrichtung der Katheterwandung (106) geweitet ist.

4. Infusionssystem (1) nach Anspruch 2, **dadurch gekennzeichnet, dass** der Schlitz (107a) in radialer Blickrichtung U-förmig in die Katheterwandung (106) eingebracht ist und einen Wandabschnitt (111a) der Katheterwandung (106) berandet, der in dem Öffnungszustand relativ zu der übrigen Katheterwandung (106) radial nach außen ausgestellt ist.

5. Infusionssystem (1) nach Anspruch 1, **dadurch gekennzeichnet, dass** dem zweiten Auslass (105) ein Ventil (112) zugeordnet ist, wobei das Ventil (112) zum fluiddruckabhängigen Öffnen und Schließen eingerichtet ist, und wobei das Ventil (112) bei einem ersten Fluiddruck (P1) geschlossen und bei einem höheren zweiten Fluiddruck (P2) geöffnet ist.

6. Infusionssystem (1) nach Anspruch 5, **dadurch gekennzeichnet, dass** das Ventil (112) dem zweiten Auslass (105) stromaufwärts vorgeschaltet ist.

7. Infusionssystem (1) nach einem der vorhergehenden Ansprüche, **dadurch gekennzeichnet, dass** der wenigstens eine erste Auslass (104) proximodistal, insbesondere zentral, zwischen wenigstens zwei zweiten Auslässen (105) angeordnet ist oder umgekehrt.

8. Infusionssystem (1) nach Anspruch 7, **dadurch gekennzeichnet, dass** eine Gruppe (G1) mit mehreren, insbesondere exakt drei, ersten Auslässen (104) proximodistal, insbesondere zentral, zwischen zwei Gruppen (G2, G2`) mit jeweils mehreren, insbesondere exakt drei, zweiten Auslässen (105) angeordnet ist oder umgekehrt.

9. Infusionssystem (1) nach einem der vorhergehenden Ansprüche, **dadurch gekennzeichnet, dass** eine Fördereinrichtung (200) und eine Steuereinrichtung (300) vorhanden sind,
wobei die Fördereinrichtung (200) fluidleitend mit dem wenigstens einen Lumen (103) des Katheters (100) verbunden und zum Fördern des Schmerzmittels (S) durch das wenigstens eine Lumen (103) mit unterschiedlichen Fluiddrücken (P1, P2) eingerichtet ist, und
wobei die Steuereinrichtung (300) mit der Fördereinrichtung (200) verbunden und zum Steuern des Fluiddrucks (P, P1, P2) und damit zum Öffnen und/oder Schließen des wenigstens einen zweiten Auslasses (105) eingerichtet ist.
